# FASCICULE DE BREVET EUROPEEN

(11) **EP 0 364 378 B1**
(45) Date de publication et mention de la délivrance du brevet: **24.02.1993**
(21) Numéro de dépôt: 89420384.3
(22) Date de dépôt: 09.10.1989
(51) Int. Cl.: C07C 37/055, C07C 37/48, C07C 39/08

(54) **Procédé de préparation de la methylhydroquinone**
Verfahren zur Herstellung von Methylhydrochinon
Process for the preparation of methyl hydroquinone

(30) Priorité: 14.10.1988 FR 8814362
(43) Date de publication de la demande: 18.04.1990
(73) Titulaire: RHONE-POULENC CHIMIE, 92408 Courbevoie Cédex (FR)
(72) Inventeur: Gubelmann, Michel, F-69006 Lyon (FR); Allandrieu, Christian, F-69100 Villeurbanne (FR)
(74) Mandataire: Dutruc-Rosset, Marie-Claude

(56) Documents cités:
- FR-A- 657 293
- GB-A- 600 839
- US-A- 3 728 408
- US-A- 4 283 572
- US-A- 4 709 102

## Description

La présente invention concerne un procédé pour la préparation de la méthylhydroquinone.

On sait que la méthylhydroquinone est un composé très intéressant comme produit intermédiaire de synthèse organique.

Il est connu de préparer des alkylphénols par chauffage des éthers alkyliques du phénol, en présence de catalyseurs tels que le gel de silice, les terres de Fuller (FR-A-675 293), l'alumine activée [GB-A-600 839] ou une zéolithe [US-A-4 709 102 ou US-A 3 728 408].

Par ailleurs, il est également connu selon [US-A-4 283 572] de transformer un éther d'alkyle et de phényle (type nonylphényléther), en alkylphénol correspondant, par chauffage en présence d'une résine échangeuse de cation du type acide sulfonique déshydraté, à une température de 60°C à 120°C.

Aucun des documents précités ne concerne la préparation de la méthylhydroquinone.

Il a été trouvé, et cela constitue l'essence de la présente invention, que l'on peut préparer la méthylhydroquinone (MeHQ) en mettant en contact avec un catalyseur acide solide, à une température de 100 à 300°C, un produit choisi parmi le paraméthoxyphénol et le paradiméthoxybenzène.

Il est préférable, selon l'invention, d'utiliser un catalyseur solide qui comporte des fonctions acides selon les définitions de Lewis ou de Bronsted. Ces catalyseurs solides peuvent être définis comme tous solides possédant une capacité échangeuse de cations qui a été partiellement ou complètement échangée par H⁺ et/ou un acide de Lewis (Mⁿ⁺ avec n > 3). Parmi ces solides, on peut citer par exemple :
- les argiles qui ont été traitées par un acide fort,
- les zéolites qui ont également subi des échanges,
- les résines du type styrène-divinylbenzène réticulées sulfonées et le NAFION^{R} (résine perfluorée et sulfonée de DUPONT DE NEMOURS),
- les oxydes acides ou amphotères dont on peut exalter la réactivité par traitement au moyen d'un acide,
- les hétéropolyacides tels que les acides phosphomolybdiques et phosphotungstiques.

Les températures de réaction peuvent être très variables dans l'intervalle d'entre environ 100 et environ 300°C ; les meilleurs résultats ont été obtenus pour des températures comprises entre 150 et 250°C.

La réaction peut s'écrire 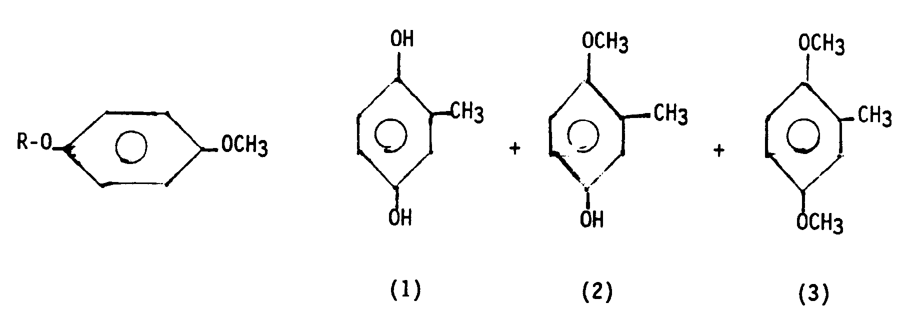
R étant CH₃ ou H
Le produit (1) étant la méthylhydroquinone qui est le produit visé et les produits (2) et (3) étant des dérivés méthylés (sur le ou les radicaux OH) de la méthylhydroquinone, ces produits (2) et (3) étant considérés ici, compte tenu de leur facilité de transformation en méthylhydroquinone, comme des "précurseurs" intéressants de cette molécule.

Lorsqu'on utilise comme produit de départ le paraméthoxyphénol on obtiendra, avec un rendement élevé, la méthylhydroquinone et relativement peu des "précurseurs" définis ci-dessus.

Lorsqu'on utilise comme produit de départ le paradiméthoxybenzène on obtiendra des proportions plus importantes de ces "précurseurs". Cependant, on notera que ce paradiméthoxybenzène est une matière première intéressante dans la mesure où il apparaît comme sous-produit à valoriser dans l'industrie chimique des polyphénols.

Si le mécanisme réactionnel peut apparaître comme relativement simple lorsqu'on utilise le paraméthoxyphénol comme produit de départ - il s'agit probablement d'une réaction de désalkylation suivie d'une réaction d'alkylation sur un carbone voisin du noyau ou d'une réaction intramoléculaire - par contre le mécanisme devient très complexe et est encore largement hypothétique donc inconnu lorsqu'on utilise le paradiméthoxybenzène comme produit de départ.

Les exemples non limitatifs ci-après illustrent l'invention.

### Exemple 1 - phase liquide

Dans un tube de verre de 50 cm³ on introduit 5,7 g (46 mmoles) de paraméthoxyphénol et 1 g de zéolite commerciale du type faujasite ultrastabilisée (US-Y de TOYO-SODA). Le tube est scellé et introduit dans une gaine métallique. L'ensemble est placé dans un four à balancement dit de "CARIUS" et chauffé à 200°C pendant 2,5 heures.

Le catalyseur solide est récupéré par filtration sur du verre fritté et lavé avec de l'acétate d'éthyle ; la solution a été analysée par chromatographie en phase gazeuse et la nature des produits a été confirmée par une analyse spectroscopique complète (infrarouge, résonance magnétique nucléaire, spectrométrie de masse).

Les résultats obtenus on montré que :
- le taux de transformation du paraméthoxyphénol utilisé était de 54 %,
- le rendement de la réaction en méthylhydroquinone plus les "précurseurs" est de 11 %,

### Exemple 2 - phase liquide

On procède comme dans l'exemple 1 mais en utilisant comme produit de départ du paradiméthoxybenzène.

Le taux de transformation est de 57 % et le rendement de 52 %.

### Exemple 3 - phase liquide

On procède comme dans l'exemple 2 mais en utilisant, au lieu de la zéolite, une argile acide commerciale (montmorillonite KSF de Sud-Chemie).

Le taux de transformation est de 58 % et le rendement de 45 %.

### Exemple 4 - phase vapeur

On introduit 1 g d'une zéolite commerciale de type faujasite ultrastabilisée (US-Y de TOYO-SODA) et 6 g de grains de quartz de 1 mm de diamètre dans un tube de quartz. Ce lit catalytique est chauffé à 400°C pendant 10 heures. Le lit est ensuite amené à 200°C.

On amène dans un tube un courant d'azote et du paradiméthoxybenzène, on fait passer sur le lit catalytique un courant gazeux constitué par 1 l par heure d'azote et 9 mmoles par heure de paradiméthoxybenzène. Le temps de contact est de 2,3 secondes.

A la sortie du réacteur les produits sont piégés puis analysés.

Le taux de transformation moyen (pendant la durée de l'essai qui a été de 3 heures) est de 23 % et le rendement de 31 %.

### Exemple 5 - phase vapeur

On procède comme dans l'exemple 4 mais en opérant à une température de 240°C.

Le taux de transformation moyen (durée de l'essai 1,8 heure) est de 19 % et le rendement de 22 %.

## Revendications

1. Procédé de préparation de la méthylhydroquinone, caractérisé en ce que l'on met en contact avec un catalyseur acide solide, à une température de 100 à 300°C, un produit choisi parmi le paraméthoxyphénol et le paradiméthoxybenzène.

2. Procédé selon la revendication 1, caractérisé en ce que la réaction est réalisée en phase liquide ou en phase vapeur.

3. Procédé selon l'une des revendications 1 ou 2, caractérisé en ce que le catalyseur acide est choisi parmi les solides possédant une capacité échangeuse de cations qui a été partiellement ou complètement échangée par H⁺ et/ou un acide de Lewis.

4. Procédé selon l'une des revendications 1 à 3, caractérisé en ce que le catalyseur acide est choisi parmi :
- les argiles qui ont été traitées par un acide fort,
- les zéolites qui ont également subi des échanges,
- les résines du type styrène-divinylbenzène réticulées sulfonées ou les résines perfluorées et sulfonées,
- les oxydes acides ou amphotères dont on peut exalter la réactivité par traitement au moyen d'un acide,
- les hétéropolyacides tels que les acides phosphomolybdiques et phosphotungstiques.

## Patentansprüche

1. Verfahren zur Herstellung von Methylhydrochinon, dadurch gekennzeichnet, daß man ein unter Paramethoxyphenol und Paradimethoxybenzol ausgewähltes Produkt bei einer Temperatur von 100 bis 300°C mit einem sauren festen Katalysator in Berührung bringt.

2. Verfahren nach Anspruch 1, dadurch gekennzeichnet, daß die Reaktion in flüssiger Phase oder in der Gasphase durchgeführt wird.

3. Verfahren nach einem der Ansprüche 1 oder 2, dadurch gekennzeichnet, daß der saure Katalysator unter den Feststoffen ausgewählt ist, die eine Kationenaustauschkapazität besitzen, die teilweise oder vollständig durch H⁺ und/oder eine Lewis-Säure ausgetauscht worden ist.

4. Verfahren nach einem der Ansprüche 1 bis 3, dadurch gekennzeichnet, daß der saure Katalysator ausgewählt ist, unter:
- den Tonen, die mit einer starken Säure behandelt wurden,
- den Zeolithen, die gleichfalls einem Austausch unterworfen wurden,
- den Harzen von Typ der vernetzten sulfonierten Styrol-Divinylbenzole oder den perfluorierten und sulfonierten Harzen,
- den sauren oder amphoteren Oxiden, deren Reaktivität durch Behandlung mit einer Säure erhöht wird,
- den Heteropolysäuren, wie den Molybdatophosphor- und Wolframatophosphorsäuren.

## Claims

1. Process for the preparation of methylhydroquinone, characterised in that a product chosen from para-methoxyphenyl and para-dimethoxybenzene is brought into contact with a solid acid catalyst at a temperature of 100 to 300°C.

2. Process according to Claim 1, characterised in that the reaction is carried out in liquid phase or in vapour phase.

3. Process according to either of Claims 1 and 2, characterised in that the acid catalyst is chosen from the solids which have a cation exchanger ability which has been partially or completely exchanged with H⁺ and/or a Lewis acid.

4. Process according to one of Claims 1 to 3, characterised in that the acid catalyst is chosen from:
- clays which have been treated with a strong acid,
- zeolites which have also been subjected to exchanges,
- resins of the sulphonated crosslinked styrene-divinylbenzene type or perfluorinated and sulphonated resins,
- acid or amphoteric oxides, the reactivity of which can be excited by treatment with an acid,
- heteropolyacids, such as phosphomolybdic and phosphotungstic acids.
